# EUROPEAN PATENT SPECIFICATION

(11) **EP 4 132 913 B1**
(45) Date of publication and mention of the grant of the patent: **05.06.2024**
(21) Application number: 21717022.4
(22) Date of filing: 01.04.2021
(51) Int. Cl.: C07D 239/48, C07D 403/12, C07D 413/12, A01N 43/54

(54) **5-HALOALKOXY-PYRIMIDINE COMPOUNDS AS HERBICIDES**
5-HALOALKOXY-PYRIMIDIN-VERBINDUNGEN ALS HERBIZIDE
COMPOSÉS DE 5-HALOALKOXY-PYRIMIDINE COMME HERBICIDES

(30) Priority: 08.04.2020 GB 202005175
(43) Date of publication of application: 15.02.2023
(73) Proprietor: Syngenta Crop Protection AG, 4058 Basel (CH)
(72) Inventor: WAILES, Jeffrey, Steven, Bracknell Berkshire RG42 6EY (GB); TATE, Joseph, Andrew, Bracknell Berkshire RG42 6EY (GB)
(74) Representative: SYNGENTA IP
(86) International application number: PCT/EP2021/058747
(87) International publication number: WO 2021/204706

(56) References cited:
- WO-A1-2015/108779
- WO-A1-2016/196606

## Description

The present invention relates to novel herbicidal compounds, to herbicidal compositions which comprise the novel compounds, and to their use for controlling weeds, in particular in crops of useful plants, or for inhibiting plant growth.

WO2015/089003, WO2015/108779 and WO2016/196606 disclose various aryloxypyrimidines and their use as herbicides. The present invention relates to novel aryloxypyrimidines.

Thus, according to the present invention there is provided a compound of Formula (I): 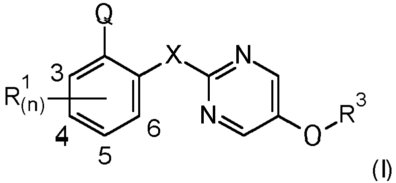 wherein
Q is selected from the group consisting of C₁-C₆haloalkyl, C₁-C₆haloalkyl-C(O)-, C₁-C₆haloalkoxy-, C₁-C₆haloalkoxyC(O)- and a 5-membered aromatic heterocyclic ring which is optionally substituted by 1 or 2 R² substituents independently selected from the group consisting of C₁-C₄alkyl, C₂-C₄alkenyl, C₂-C₄alkynyl, cyclopropyl, C₁-C₄haloalkyl, C₁-C₂alkoxy-, C₁-C₂haloalkoxy-, halogen, -C(O)C₁-C₄alkyl, NO₂, -CH₂CN, -CN and -S(O)ₚC₁-C₄alkyl;
each R¹ is independently selected from the group consisting of halogen, -CN, nitro, C₁-C₄alkyl, C₂-C₄alkenyl, C₂-C₄alkynyl, C₁-C₄haloalkyl, C₁-C₄alkoxy-, C₁-C₄haloalkoxy- and -S(O)ₚC₁-C₄alkyl;
R³ is C₁-C₂ haloalkyl;
X = O or S(O)ₚ;
n = 0, 1 or 2; and
p = 0, 1 or 2.
C₁-C₄alkyl- includes, for example, methyl (Me, CH₃), ethyl (Et, C₂H₅), *n*-propyl (*n*-Pr), isopropyl (*i*-Pr), *n*-butyl (*n*-Bu), isobutyl (*i*-Bu), *sec*-butyl and *tert*-butyl (*t*-Bu). C₁-C₂alkyl is methyl (Me, CH₃) or ethyl (Et, C₂H₅).

Halogen (or halo) includes, for example, fluorine, chlorine, bromine or iodine. The same correspondingly applies to halogen in the context of other definitions, such as haloalkyl.

C₁-C₆haloalkyl- includes, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoropropyl and 2,2,2-trichloroethyl, heptafluoro-n-propyl and perfluoro-n-hexyl. C₁-C₄haloalkyl- and C₁-C₂haloalkyl include, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, or 1,1-difluoro-2,2,2-trichloroethyl.

C₁-C₄alkoxy and C₁-C₂alkoxy includes, for example, methoxy and ethoxy.

C₁-C₆haloalkoxy- and C₁-C₄haloalkoxy- include, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₂-C₄alkenyl- includes, for example, -CH=CH₂ (vinyl) and -CH₂-CH=CH₂ (allyl).

C₂-C₄alkynyl- refers to a straight or branched hydrocarbon chain radical group consisting solely of carbon and hydrogen atoms, containing at least one triple bond, having from two to four carbon atoms, and which is attached to the rest of the molecule by a single bond. Examples of C₂₋C₄alkynyl include, but are not limited to, prop-1-ynyl, propargyl (prop-2-ynyl), and but-1-ynyl.

C₁-C₄alkyl-S- (alkylthio) includes, for example, methylthio, ethylthio, propylthio, isopropylthio, n-butylthio, isobutylthio, sec-butylthio or tert-butylthio, preferably methylthio or ethylthio.

C₁-C₄alkyl-S(O)- (alkylsulfinyl) includes, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₄alkyl-S(O)₂- (alkylsulfonyl) includes, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In one preferred embodiment of the present invention there is provided a compound of Formula (I) wherein Q is C₁-C₆fluoroalkyl.

In another preferred embodiment of the present invention there is provided a compound of Formula (I) wherein Q is C₁-C₆fluoroalkyl-C(O)-.

In another preferred embodiment of the present invention there is provided a compound of Formula (I) wherein Q is C₁-C₆haloalkoxy- (e.g CF₃CH₂CH₂O-).

In another preferred embodiment of the present invention there is provided a compound of Formula (I) wherein Q is C₁-C₆haloalkoxyC(O)- (e.g CF₃CH₂OC(O)-).

In another embodiment of the present invention there is provided a compound of Formula (I) wherein Q is a 5-membered aromatic heterocyclic ring which is optionally substituted by 1 or 2 R² substituents independently selected from the group consisting of C₁-C₄alkyl, C₂-C₄alkenyl, C₂-C₄alkynyl, cyclopropyl, C₁-C₄haloalkyl, C₁-C₂alkoxy-, C₁-C₂haloalkoxy-, halogen, -C(O)C₁-C₄alkyl, NO₂, -CH₂CN, -CN and - S(O)ₚC₁-C₄alkyl;

In a preferred embodiment of the present invention there is provided a compound of Formula (I) wherein Q is selected from the group consisting of: 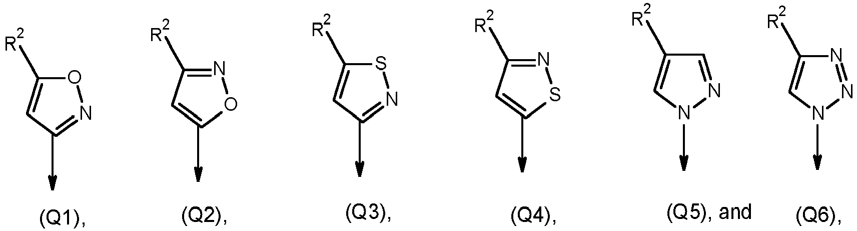 wherein R² is selected from the group consisting of hydrogen, C₁-C₄alkyl, C₂-C₄alkenyl, C₂-C₄alkynyl, cyclopropyl, C₁-C₂haloalkyl, C₁-C₂alkoxy-, C₁-C₂haloalkoxy-, halogen, -C(O)C₁-C₄alkyl, NO₂, -CH₂CN, -CN and -S(O)ₚC₁-C₄alkyl.

In a more preferred embodiment of the present invention there is provided a compound of Formula (I), wherein Q is selected from the group consisting of Q1, Q2 and Q5, more preferably Q2.

In a preferred embodiment R² is C₁-C₂haloalkyl. In a more preferred embodiment R³ is difluoromethyl or trifluoromethyl.

In one embodiment of the present invention, n is 1 and R¹ is selected from the group consisting of fluoro, chloro, bromo and CN. In a more preferred embodiment n is 1 and the R¹ substituent is present in the 3-position.

In another embodiment of the present invention there is provided a compound of Formula (I), wherein R³ is CF₃ or CHF₂.

Thus, in a preferred embodiment of the present invention the compound of Formula (I) is a compound of Formula (Ia): 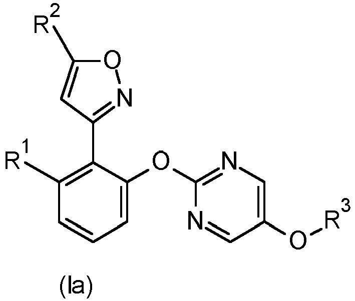 wherein R¹ is selected from the group consisting of hydrogen, fluoro, chloro and bromo, R² is CF₃ or -CHF₂, R³ is CF₃ or -CHF₂.

In another preferred embodiment of the present invention the compound of Formula (I) is a compound of Formula (Ib): 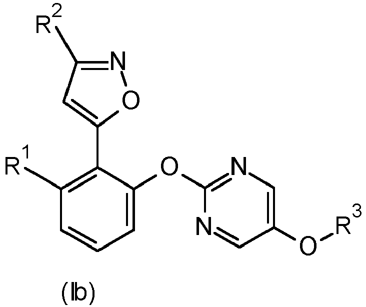 wherein R¹ is selected from the group consisting of hydrogen, fluoro, chloro and bromo, R² is CF₃ or -CHF₂, R³ is CF₃ or -CHF₂.

In another preferred embodiment of the present invention the compound of Formula (I) is a compound of Formula (Ic): 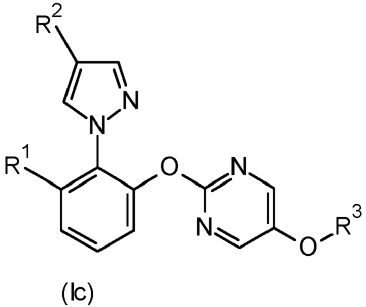 wherein R¹ is selected from the group consisting of hydrogen, fluoro, chloro and bromo, R² is CF₃ or -CHF₂, R³ is CF₃ or -CHF₂.

Compounds of Formula (I) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also provides agronomically acceptable salts of compounds of Formula (I). Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred.

The compounds of Formula (I) according to the invention can be used as herbicides by themselves, but they are generally formulated into herbicidal compositions using formulation adjuvants, such as carriers, solvents and surface-active agents (SAA). Thus, the present invention further provides a herbicidal composition comprising a herbicidal compound according to the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

As outlined above, the compounds of the present invention may contain an asymmetric centre. As such, the compound of the present invention may be present in the composition as a racemic mixture of the two enantiomers. Alternatively, the compound of the present invention may be present in an enantiomer enriched form.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butyl naphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates.

Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, ethyl 2-[[3-[2-chloro-4-fluoro-5-[3-methyl-2,6-dioxo-4-(trifluoromethyl)pyrimidin-1-yl]phenoxy]-2-pyridyl]oxy]acetate, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethyl-cyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione, 4-[6-cyclopropyl-2-(3,4-dimethoxyphenyl)-3-oxo-pyridazine-4-carbonyl]-2,2,6,6-tetramethyl-tetrahydropyran-3,5-dione, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide and ethyl 2-[[3-[[3-chloro-5-fluoro-6-[3-methyl-2,6-dioxo-4-(trifluoromethyl)pyrimidin-1-yl]-2-pyridyl]oxy]acetate.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or metcamifen.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus, said method comprising application to the locus of a weed controlling amount of a composition comprising a compound of Formula (I). Moreover, the present invention may further provide a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. It is noted that the compounds of the present invention show a much-improved selectivity compared to known, structurally similar compounds. Generally, the plants to be controlled are unwanted plants (weeds). 'Locus' means the area in which the plants are growing or will grow. The application may be applied to the locus pre-emergence and/or post-emergence of the crop plant. Some crop plants may be inherently tolerant to herbicidal effects of compounds of Formula (I). Preferred crop plants include maize, wheat, barley, soya and rice.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to other herbicides or classes of herbicides (e.g. ALS-, GS-, EPSPS-, PPO-, HPPD-, -PDS and ACCase-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compositions can be used to control unwanted plants (collectively, 'weeds'). The weeds to be controlled may be both monocotyledonous species, for example *Agrostis, Alopecurus, Avena, Brachiaria, Bromus, Concerns, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

In a further aspect of the present invention there is provided the use of a compound of Formula (I) as defined herein as a herbicide.

The compounds of the present invention can be prepared according to the following schemes.

Typical abbreviations used throughout are as follows:
d = doublet
DCM = dichloromethane
DMF = *N,N*-dimethylformamide
Et₂O = diethyl ether
EtOAc = ethyl acetate
IPA = isopropyl alcohol
LED = light emitting diode
Me = Methyl
q = quartet
RT = room temperature
s = singlet
t = triplet

### Processes for preparation of compounds, e.g. compounds of formula (I)

Processes for preparation of compounds, e.g. a compound of formula (I) (which optionally can be an agrochemically acceptable salt thereof), are now described, and form further aspects of the present invention.

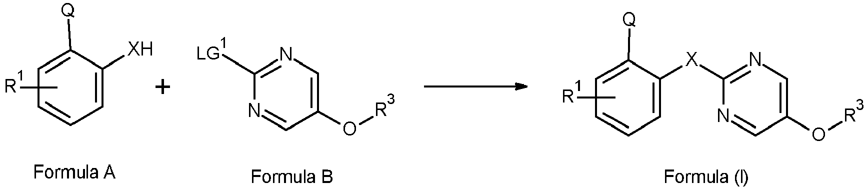

A compound of Formula (I) may be prepared from a compound of Formula A by reaction with a compound of Formula B (where LG¹ represents a suitable leaving group such as F, Cl, Br or SO₂Me), optionally in the presence of a suitable base and in a suitable solvent at a suitable reaction temperature. Suitable bases may include K₂CO₃ or Cs₂CO₃. Suitable solvents may include DMF or CH₃CN. Suitable reaction temperatures are between room temperature and 100°C. Compounds of Formula A may be prepared by methods known in the literature.

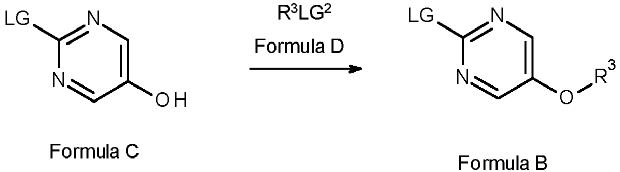

A compound of Formula B may be prepared from a compound of Formula C via an alkylation reaction with a compound of Formula D (where LG² represents a suitable leaving group such as C!, Br, I, OSO₂Me or OSO₂CF₃) optionally in the presence of a suitable base and in a suitable solvent. Suitable bases may include K₂CO₃ or Cs₂CO₃. Suitable solvents may include DMF or CH₃CN. Compounds of Formula C and of Formula D are commercially available or may be prepared by methods known in the literature.

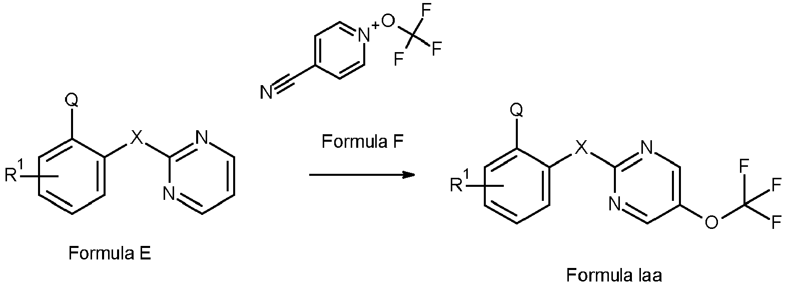

In an alternative method, a compound of Formula laa (a compound of Formula I where R³ = CF₃) may be prepared from a compound of Formula E by reaction with a compound of Formula F in the presence of a suitable catalyst and in a suitable solvent, optionally in the presence of a suitable light source. Suitable catalysts may include tris(2,2'-bipyridine)ruthenium(II) hexafluorophosphate. Suitable solvents may include CH₃CN. Suitable light sources may include blue LEDs. Compounds of Formula F may be prepared by methods known in the literature.

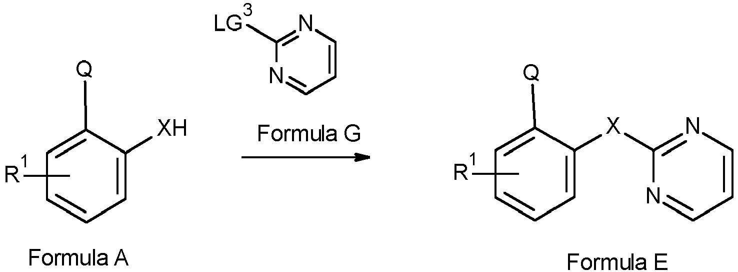

A compound of Formula E may be prepared from a compound of Formula A by reaction with a compound of Formula G (where LG³ represents a suitable leaving group such as F, Cl, Br or SO₂Me) optionally in the presence of a suitable base and in a suitable solvent. Suitable bases may include K₂CO₃ or Cs₂CO₃. Suitable solvents may include DMF or CH₃CN. Suitable reaction temperatures are between room temperature and 100°C. Compounds of Formula A may be prepared according to methods known in the literature. Compounds of Formula G are commercially available or may be prepared according methods known in the literature.

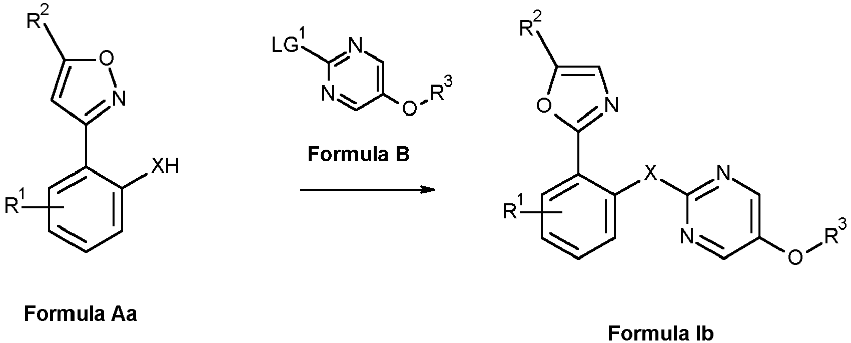

In a yet further alternative method, a compound of Formula Ib (a compound of Formula I where Q = 2-oxazole and R² is a electron withdrawing group) may be prepared from a compound of Formula Aa ( a compound of Formula A where Q = 3-isoxazole and R² is an electron withdrawing group) by reaction with a compound of Formula B (where LG¹ represents a suitable leaving group such as F, Cl, Br or SO₂Me), optionally in the presence of a suitable base and in a suitable solvent at a suitable reaction temperature. Suitable bases may include K₂CO₃ or Cs₂CO₃. Suitable solvents may include DMF or CH₃CN. Suitable reaction temperatures are between room temperature and 100°C.

The following non-limiting examples provide specific synthesis methods for representative compounds of the present invention, as referred to in Table 1 below.

2-[3-(difluoromethyl)isoxazol-5-yl]-3-fluoro-phenol was prepared according to procedures described in WO2015/108779. 1-(trifluoromethoxy)pyridin-1-ium-4-carbonitrile was prepared according to the procedure described in Angew.Chem.Int.Ed. 2018, 57,13784-13789. 1-(2-chloro-6-hydroxy-phenyl)-4,4,4-trifluoro-butan-1-one and 3-chloro-2-(4,4,4-trifluorobutyl)phenol were prepared according to procedures in WO2016/196606.

### Example 1: Synthesis of 5-[2-[5-(difluoromethoxy)pyrimidin-2-yl]oxy-6-fluorophenyl]-3-(difluoromethyl)isoxazole (Compound 1.001)

### Step 1: Synthesis of 5-[2-[5-(difluoromethoxy)pyrimidin-2-yl]oxy-6-fluoro-phenyl]-3-(difluoromethyl)isoxazole (1.001)

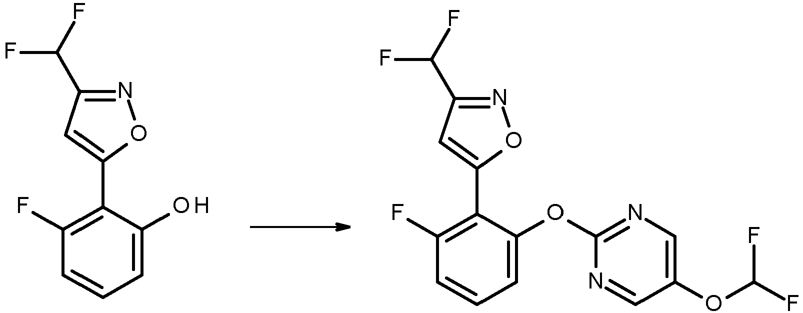

A solution of 2-[3-(difluoromethyl)isoxazol-5-yl]-3-fluoro-phenol (0.2 g, 0.87 mmol), 2-chloro-5-(difluoromethoxy)pyrimidine (0.189 g, 1.05 mmol) and K₂CO₃ (0.24 g, 1.75 mmol) in DMF (2.6 mL) was heated at 80 °C for 3h. The reaction was allowed to cool to RT, diluted with water and extracted with Et₂O (x3). The combined organic extracts were washed with brine, dried over Na₂SO₄ and evaporated to dryness under reduced pressure.

The crude product was purified by flash chromatography on silica gel using a gradient of 0-70% EtOAc / cyclohexane as eluent.

The still crude product was dissolved in DCM and washed with 2 portions of 2M NaOH. The organic portion was dried over Na₂SO₄ and evaporated to dryness under reduced pressure to give the desired product (0.181 g, 56%) as a colourless gum.

¹H NMR (400 MHz, CDCl₃) δ 8.40 (s, 2H), 7.55 (td, 1H), 7.23-7.14 (m, 2H), 6.88 (s, 1H), 6.75 (t, 1H), 6.52 (t, 1H).

### Example 2: Synthesis of 3-(difluoromethyl)-5-[2-fluoro-6-[5-(trifluoromethoxy)pyrimidin-2-yl]oxy-phenyl]isoxazole (Compound 1.002)

### Step 1: Synthesis of 3-(difluoromethyl)-5-(2-fluoro-6-pyrimidin-2-yloxy-phenyl)isoxazole

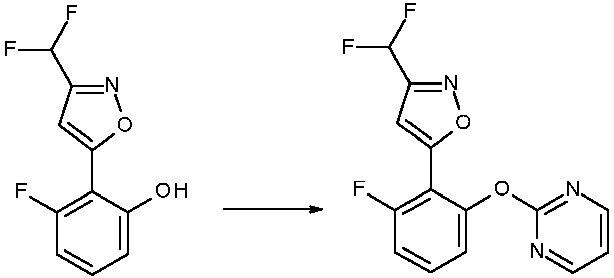

A solution of 2-[3-(difluoromethyl)isoxazol-5-yl]-3-fluoro-phenol (0.50 g, 2.18 mmol), 2-chloropyrimidine (0.30 g, 2.62 mmol) and K₂CO₃ (0.603 g, 4.36 mmol) in DMF (6.50 mL) was heated at 80 °C for 3 h. The reaction was allowed to cool to RT, diluted with water and extracted with Et₂O (x3). The combined organic extracts were washed with brine, dried over MgSO₄ and evaporated to dryness. The crude product was purified by flash chromatography on silica gel using a gradient of 0-60% EtOAc/cyclohexane as eluent gave the desired product (0.497 g, 74%) as an off white solid.

¹H NMR (400 MHz, CDCl₃) δ 8.54 (d, 2H), 7.55 (td, 1H), 7.22-7.15 (m, 2H), 7.06 (t, 1H), 6.86-6.60 (m, 2H).

### Step 2: Synthesis of 3-(difluoromethyl)-5-[2-fluoro-6-[5-(trifluoromethoxy)pyrimidin-2-yl]oxy-phenyl]isoxazole (Cmpound 1.002)

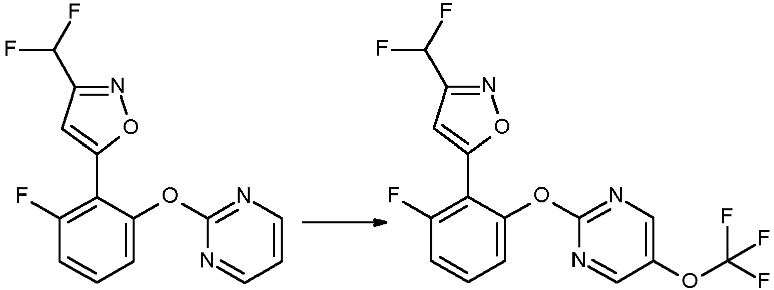

A dried pressure vial was charged with 1-(trifluoromethoxy)pyridin-1-ium-4-carbonitrile (0.11 g, 0.58 mmol), 3-(difluoromethyl)-5-(2-fluoro-6-pyrimidin-2-yloxy-phenyl)isoxazole (0.357 g, 1.16 mmol) and tris(2,2'-bipyridine)ruthenium(II) hexafluorophosphate (0.051 g, 0.058 mmol). The flask was flushed with nitrogen, then CH₃CN (11.6 mL) was added, the vial sealed and the reaction was stirred at RT for 1 h with exposure to blue LEDs in a photoreactor. The reaction mixture was diluted with H₂O and extracted with Et₂O (x3). The combined organic extracts were washed with brine, dried over Na₂SO₄ and evaporated to dryness under reduced pressure. The crude product was purified by flash column chromatography on silica gel using a gradient of 0-50% EtOAc/cyclohexane as eluent to give the desired product (0.014 g, 6%) as a colourless gum.
¹H NMR (400 MHz, CDCl₃) δ 8.46 (s, 2H), 7.57 (td, 1H), 7.22 (td, 1H), 7.16 (d, 1H), 6.89 (s, 1H), 6.75 (t, 1H)

### Example 3: Synthesis of 1-[2-chloro-6-[5-(difluoromethoxy)pyrimidin-2-yl]oxyphenyl]-4,4,4-trifluoro-butan-1-one (1.027)

### Step 1: Synthesis of 1-[2-chloro-6-[5-(difluoromethoxy)pyrimidin-2-yl]oxy-phenyl]-4,4,4-trifluoro-butan-1-one (1.027)

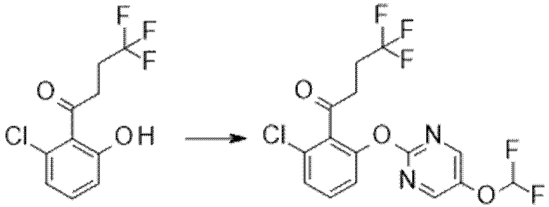

A mixture of 1-(2-chloro-6-hydroxy-phenyl)-4,4,4-trifluoro-butan-1-one (55 mg, 0.218 mmol), 5-(difluoromethoxy)-2-methylsulfonyl-pyrimidine (66 mg, 0.294 mmol) and potassium carbonate (26 mg, 0.261 mmol) in IPA (1.0 mL) was heated at 50°C for an hour. The reaction was cooled, diluted with dilute HCl and extracted with Et₂O. The combined organic extracts were washed with brine, dried over MgSO₄ and evaporated to dryness. The crude product was purified by flash column chromatography on silica gel using a gradient of EtOAc in cyclohexane to give the desired product (58 mg, 68%) as a colourless oil.
¹H NMR (400 MHz, CDCl₃) δ 8.43 (s, 2H), 7.43 (t, 1H), 7.35 (dd, 1H), 7.15 (dd, 1H), 6.55 (t, 1H), 3.18- 3.04 (m, 2H), 2.61-2.41 (m, 2H)

### Example 4: Synthesis of 2-[3-chloro-2-(4,4,4-trifluorobutyl)phenoxy]-5-(difluoromethoxy)pyrimidine (1.023)

### Step 1: Synthesis of 2-[3-chloro-2-(4,4,4-trifluorobutyl)phenoxy]-5-(difluoromethoxy)pyrimidine (1.023)

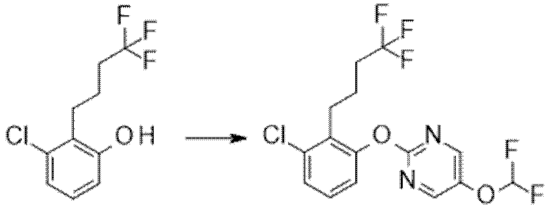

A mixture of 3-chloro-2-(4,4,4-trifluorobutyl)phenol (80 mg, 0.335 mmol), 2-chloro-5-(difluoromethoxy)pyrimidine (73 mg, 0.402 mmol) and potassium carbonate (603 mg, 4.36 mmol) in DMF (0.8 mL) was heated at 50°C for 2 hours. The reaction was cooled, diluted with 2M HCl and extracted with Et₂O. The combined organic extracts were washed with brine, dried over MgSO₄ and evaporated to dryness. The crude product was purified by reverse-phase chromatography to give the desired product (108 mg, 84%) as a colourless gum.

¹H NMR (400 MHz, CDCl₃) δ 8.44 (s, 2H), 7.33 (dd, 1H), 7.24 (t, 1H), 7.04 (dd, 1H), 6.55 (t, 1H), 2.81 (t, 2H), 2.19-2.03 (m, 2H), 1.88-1.77 (m, 2H)

### Example 5: Synthesis of 3-(difluoromethyl)-5-[2-fluoro-6-[5-(2,2,2-trifluoroethoxy)pyrimidin-2-yl]oxy-phenyl]isoxazole (1.030)

### Step 1: Synthesis of 2-methylsulfanyl-5-(2,2,2-trifluoroethoxy)pyrimidine

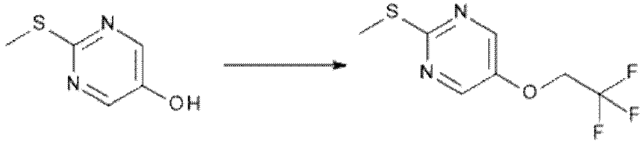

To a solution of 2-methylsulfanylpyrimidin-5-ol (0.200 g, 1.41 mmol) in acetonitrile (2 mL) was added potassium carbonate (0.389g, 2.81 mmol) and 2,2,2-trifluoroethyl trifluoromethanesulfonate (0.243 mL, 1.69 mmol) and stirred at RT overnight. The reaction mixture was diluted with water and extracted with EtOAc (3x). The combined organic extracts were washed with brine, dried over MgSO₄ and evaporated to dryness under reduced pressure. The crude product was purified by flash chromatography on silica gel using a gradient of 0-50% EtOAc in cyclohexane as eluent to give the desired product (0.224g, 71%) as a white solid.
¹H NMR (400 MHz, CDCl₃) δ 8.33 (s, 2H), 4.40 (q, 2H), 2.56 (s, 3H)

### Step 2: Synthesis of 2-methylsulfonyl-5-(2,2,2-trifluoroethoxy)pyrimidine

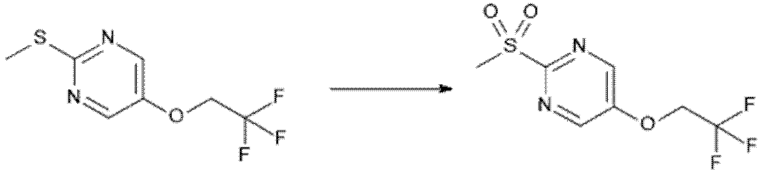

To a solution of 2-methylsulfanyl-5-(2,2,2-trifluoroethoxy)pyrimidine (0.224 g, 0.999 mmol) in dichloromethane (10 mL) at 0°C was added 3-chloroperbenzoic acid (0.575g, 2.50 mmol). The reaction was allowed to warm to RT over 5 hours then quenched with 20% sodium metabisulfite solution. The phases were separated and the organic phase washed with two portions of sat. aq. NaHCOs solution. The organic phase was dried over MgSO₄ and evaporated to dryness under reduced pressure to give the desired product (0.226g, 88%) as a white solid.
¹H NMR (400 MHz, CDCl₃) 8.63 (s, 2H), 4.60 (q, 2H), 3.35 (s, 3H)

### Step 3: Synthesis of 3-(difluoromethyl)-5-[2-fluoro-6-[5-(2,2,2-trifluoroethoxy)pyrimidin -2-yl]oxy-phenyl]isoxazole (1.030)

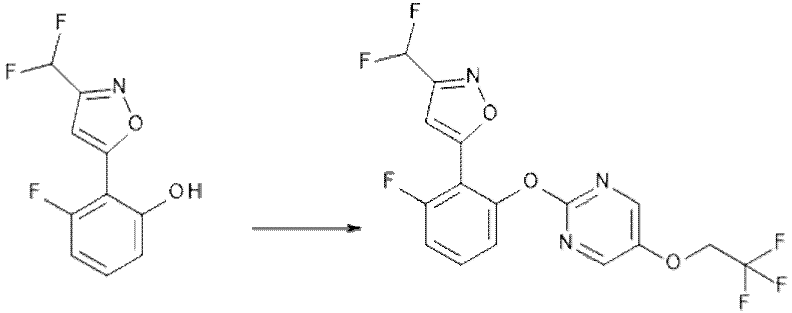

To a solution of 2-[3-(difluoromethyl)isoxazol-5-yl]-3-fluoro-phenol (0.069g, 0.30 mmol) in acetonitrile (1 mL) at RT was added potassium carbonate (0.076g, 0.55 mmol) and 2-methylsulfonyl-5-(2,2,2-trifluoroethoxy)pyrimidine (0.070 g, 0.27 mmol). The reaction was heated at 75°C overnight, allowed to cool to RT, diluted with water and extracted with EtOAc (x3). The combined organic extracts were washed with brine, dried over MgSO₄ and evaporated to dryness under reduced pressure. The crude product was purified by flash chromatography on silica gel using a gradient of 0-25% EtOAc/cyclohexane as eluent to give the desired product (0.086g, 78%) as a colourless oil.
¹H NMR (400 MHz, CDCl₃) δ 8.27 (s, 2H), 7.54 (dt, 1H), 7.19 (ddd, 1H), 7.13 (td, 1H), 6.86 (d, 1H), 6.74 (t, 1H), 4.39 (q, 2H)

**Table 1 - Examples of herbicidal compounds of the present invention.**

| 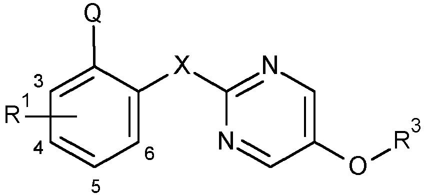 | | | | | |
|---|---|---|---|---|---|
| **Compound** | **Q** | **R¹** | **X** | **R³** | **¹H NMR (400MHz, CDCl₃ unless stated)** |
| 1.001 | 5-(3-difluoromethyl)-isoxazole | 3-F | O | CHF₂ | 8.40 (s, 2H), 7.55 (td, 1H), 7.23-7.14 (m, 2H), 6.88 (s, 1H), 6.75 (t, 1H), 6.52 (t, 1H) |
| 1.002 | 5-(3-difluoromethyl)-isoxazole | 3-F | O | CF₃ | 8.46 (s, 2H), 7.57 (td, 1H), 7.22 (td, 1H), 7.16 (d, 1H), 6.89 (s, 1H), 6.75 (t, 1H) |
| 1.003 | 5-(3-difluoromethyl)-isoxazole | - | O | CHF₂ | 8.43 (s, 2H), 8.07 (dd, 1H), 7.57 (ddd, 1H), 7.44 (dt, 1H), 7.32 (dd, 1H), 6.90 (s, 1H), 6.75 (t, 1H), 6.54 (t, 1H) |
| 1.004 | 5-(3-difluoromethyl)-isoxazole | 3-Cl | O | CHF₂ | 8.40 (s, 2H), 7.53 (t, 1H), 7.49 (dd, 1H) 7.24 (dd, 1H), 6.74 (t, 1H), 6.70 (s, 1H), 6.53 (t, 1H) |
| 1.005 | 5-(3-difluoromethyl)-isoxazole | 3-Br | O | CHF₂ | 8.38 (s, 2H), 7.67 (dd, 1H), 7.46 (t, 1H), 7.27 (dd, 1H), 6.73 (t, 1H), 6.65 (s, 1H), 6.52 (t, 1H) |
| 1.006 | 5-(3-difluoromethyl)-isoxazole | 3-CN | O | CHF₂ | 8.41 (s, 2H), 7.80 (dd, 1H), 7.70 (t, 1H), 7.59 (dd, 1H), 7.00 (s, 1H), 6.77 (t, 1H), 6.55 (t, 1H) |
| 1.007 | 3-(5-trifluoromethyl)-isoxazole | - | O | CHF₂ | |
| 1.008 | 3-(5-trifluoromethyl)-isoxazole | 3-F | O | CHF₂ | 8.40 (s, 2H), 7.56-7.48 (1H, m), 7.20-7.05 (2H, m), 6.98 (s, 1H), 6.47 (t, 1H) |
| 1.009 | 3-(5-trifluoromethyl)-isoxazole | 3-CI | O | CHF₂ | |
| 1.010 | 3-(5-trifluoromethyl)-isoxazole | 3-Br | O | CHF₂ | |
| 1.011 | 3-(5-trifluoromethyl)-isoxazole | 3-CN | O | CHF₂ | |
| 1.012 | 4-(trifluoromethyl)-pyrazol-1-yl | - | O | CHF₂ | 8.32 (s, 2H), 8.18 (s, 1H), 7.80-7.71 (m, 2H), 7.51-7.39 (m, 2H), 7.35 (d, 1H), 6.49 (t, 1H) |
| 1.013 | 4-(trifluoromethyl)-pyrazol-1-yl | 3-F | O | CHF₂ | |
| 1.014 | 4-(trifluoromethyl)-pyrazol-1-yl | 3-Cl | O | CHF₂ | |
| 1.015 | 4-(trifluoromethyl)-pyrazol-1-yl | 3-Br | O | CHF₂ | |
| 1.016 | 4-(trifluoromethyl)-pyrazol-1-yl | 3-CN | O | CHF₂ | |
| 1.017 | 4-(difluoromethyl)-triazol-1-yl | - | O | CHF₂ | 8.35 (s, 2H), 8.28 (s, 1H), 7.87 (d, 1H), 7.59 (t, 1H), 7.49 (t, 1H), 7.40 (d, 1H), 6.83 (t, 1H), 6.52 (t, 1H) |
| 1.018 | 4-(difluoromethyl)-triazol-1-yl | 3-F | O | CHF₂ | |
| 1.019 | 4-(difluoromethyl)-triazol-1-yl | 3-Cl | O | CHF₂ | |
| 1.020 | 4-(difluoromethyl)-triazol-1-yl | 3-Br | O | CHF₂ | |
| 1.021 | 4-(difluoromethyl)-triazol-1-yl | 3-CN | O | CHF₂ | |
| 1.022 | -CH₂CH₂CH₂CH₂CF₃ | 3-F | O | CHF₂ | |
| 1.023 | -CH₂CH₂CH₂CF₃ | 3-CI | O | CHF₂ | 8.44 (s, 2H), 7.33 (dd, 1H), 7.24 (t, 1H), 7.04 (dd, 1H), 6.55 (t, 1H), 2.81 (t, 2H), 2.19-2.03 (m, 2H), 1.88-1.77 (m, 2H) |
| 1.024 | -CH₂CH₂CH₂CF₃ | 3-Br | O | CHF₂ | 8.45 (s, 2H), 7.51 (dd, 1H), 7.18 (t, 1H), 7.08 (dd, 1H), 6.55 (t, 1H), 2.83 (t, 2H), 2.21-2.01 (m, 2H), 1.90-1.70 (m, 2H) |
| 1.025 | -CH₂CH₂CH₂CF₃ | 3-CN | O | CHF₂ | 8.45 (s, 2H), 7.60 (dd, 1H), 7.41 (t, 1H), 7.37 (dd, 1H), 6.57 (t, 1H), 2.91 (t, 2H), 2.23-2.04 (m, 2H), 1.94-1.82 (m, 2H) |
| 1.026 | -C(O)CH₂CH₂CF₃ | 3-F | O | CHF₂ | |
| 1.027 | -C(O)CH₂CH₂CF₃ | 3-Cl | O | CHF₂ | 8.43 (s, 2H), 7.43 (t, 1H), 7.35 (dd, 1H), 7.15 (dd, 1H), 6.55 (t, 1H), 3.18-3.04 (m, 2H), 2.61- 2.41 (m, 2H) |
| 1.028 | -C(O)CH₂CH₂CF₃ | 3-Br | O | CHF₂ | 8.43 (s, 2H), 7.52 (dd, 1H), 7.36 (t, 1H), 7.19 (dd, 1H), 6.55 (t, 1H), 3.16-3.09 (m, 2H), 2.59-2.46 (m, 2H) |
| 1.029 | -C(O)CH₂CH₂CF₃ | 3-CN | O | CHF₂ | |
| 1.030 | 5-(3-difluoromethyl)-isoxazole | 3-F | O | CH₂CF₃ | 8.27 (s, 2H), 7.54 (dt, 1H), 7.19 (ddd, 1H), 7.13 (td, 1H), 6.86 (d, 1H), 6.74 (t, 1H), 4.39 (q, 2H) |
| 1.031 | 5-(3-difluoromethyl)-isoxazole | 3-Br | O | CH₂CF₃ | 8.24 (s, 2H), 7.65 (dd, 1H), 7.45 (t, 1H), 7.24 (d, 1H), 6.73 (t, 1H), 6.64 (s, 1H), 4.38 (q, 2H) |
| 1.032 | 5-(3-difluoromethyl)-isoxazole | 3-CN | O | CH₂CF₃ | 8.27 (m, 2H), 7.78 (dd, 1H), 7.68 (t, 1H), 7.56 (dd, 1H), 6.99 (s, 1H), 6.77 (t, 1H), 4.40 (q, 2H) |
| 1.033 | 2-(5-trifluoromethyl)-isoxazole | 3-F | O | CHF₂ | 8.40 (s, 2H), 7.64-7.55 (m 1H), 7.46 (s, 1H), 7.27-7.16 (m, 2H), 6.50 (t, 1H) |

### Biological Examples

Seeds of a variety of test species are sown in standard soil in pots, *Amaranthus retoflexus* (AMARE), *Abutilon theophrasti* (ABUTH), *Setaria faberi* (SETFA), *Echinochloa crus-galli* (ECHCG) and *Ipomoea hederacea* (IPOHE). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 1000 g/ha unless otherwise stated. The test plants are then grown in a glasshouse under controlled conditions in a glasshouse (at 24/16°C, day/night; 14 hours light; 65 % humidity) and watered twice daily. After 13 days for pre and post-emergence, the test is evaluated for the percentage damage caused to the plant. The biological activities (% phytotoxicity) are shown in Tables B1 and B2 below.

**Table B1. Post-emergence activity.**

| Compound | Rate | AMARE | ABUTH | SETFA | ECHCG | IPOHE |
|---|---|---|---|---|---|---|
| 1.001 | 250 | 4 | 3 | 4 | NT | 3 |
| 1.002 | 250 | 4 | 4 | 4 | 4 | 3 |
| 1.003 | 250 | 4 | 4 | 3 | 3 | 4 |
| 1.004 | 250 | 4 | 4 | 4 | 4 | 4 |
| 1.005 | 250 | 4 | 4 | 4 | 3 | 5 |
| 1.006 | 250 | 4 | 4 | 3 | 3 | 4 |
| 1.023 | 250 | 1 | 1 | 1 | 1 | 1 |
| 1.024 | 250 | 2 | 1 | 1 | 1 | 1 |
| 1.025 | 250 | 2 | 1 | 1 | 1 | 1 |
| 1.027 | 250 | 4 | 3 | 1 | 1 | 3 |
| 1.028 | 250 | 5 | 2 | 1 | 1 | 2 |

**Table B2. Pre-emergence activity.**

| Compound | Rate | AMARE | ABUTH | SETFA | ECHCG | IPOHE |
|---|---|---|---|---|---|---|
| 1.001 | 250 | 5 | 5 | 5 | NT | 5 |
| 1.002 | 250 | 5 | 5 | 5 | 4 | 4 |
| 1.003 | 250 | 5 | 4 | 4 | 2 | 3 |
| 1.004 | 250 | 5 | 4 | 5 | 4 | 5 |
| 1.005 | 250 | 5 | 3 | 5 | 3 | 4 |
| 1.006 | 250 | 5 | 5 | 5 | 3 | 3 |
| 1.023 | 250 | 1 | 1 | 1 | 1 | 1 |
| 1.024 | 250 | 2 | 1 | 1 | 1 | 1 |
| 1.025 | 250 | 1 | 1 | 1 | 1 | 1 |
| 1.027 | 250 | 4 | 3 | 4 | 2 | 1 |
| 1.028 | 250 | 5 | 2 | 5 | 1 | 1 |

| | | | | | | |
|---|---|---|---|---|---|---|
| NT = Not tested. | | | | | | |

### Comparative Tests.

Seeds of test species were sown in standard soil in pots. After cultivation for 10 days (post-emergence) under controlled conditions in a glasshouse (at 24/16°C, day/night; 14 hours light; 65 % humidity), the plants were sprayed with an aqueous spray solution derived from the formulation of the technical active ingredient in 0.6 ml acetone and 45 ml formulation solution containing 10.6% Emulsogen EL (Registry number 61791-12-6), 42.2% N-methyl pyrrolidone, 42.2% dipropylene glycol monomethyl ether (CAS RN 34590-94-8) and 0.2 % X-77 (CAS RN 11097-66-8). The test plants were then grown in a glasshouse under controlled conditions in a glasshouse (at 24/16°C, day/night; 14 hours light; 65 % humidity) and watered twice daily. After 14 days, the test was evaluated (100 = total damage to plant; 0 = no damage to plant).

### Test species:

Weeds: *Ipomea hederacea (IPOHE)*
Crops: Zea *mays (ZEAMX)*

**Table B3a**

| **Compound** | **Rate (g/ha)** | **ZEAMX** | **IPOHE** |
|---|---|---|---|
| 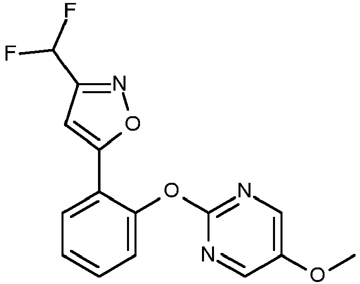 Compound 213 from WO2015/108779 | 500 | 20 | 50 |
| | 250 | 0 | 40 |
| | 125 | 0 | 30 |
| | 60 | 0 | 20 |
| | 8 | 0 | 10 |
| | 2 | 0 | 0 |
| 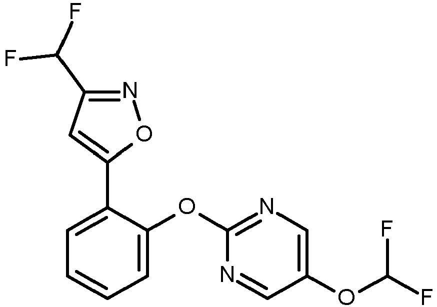 Compound 1.003 of the present invention | 500 | 10 | 90 |
| | 250 | 0 | 70 |
| | 125 | 0 | 70 |
| | 60 | 0 | 30 |
| | 8 | 0 | 20 |
| | 2 | 0 | 10 |

**Table B3b**

| **Compound** | **Rate (g/ha)** | **ZEAMX** | **IPOHE** |
|---|---|---|---|
| 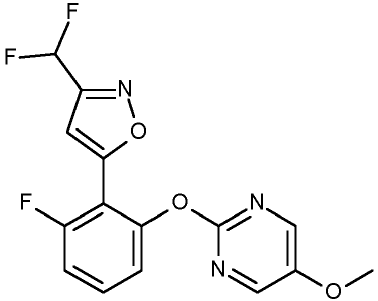 Comparator compound of the type disclosed in WO2015/108779 | 500 | 40 | 70 |
| | 250 | 10 | 60 |
| | 125 | 10 | 50 |
| | 60 | 10 | 20 |
| | 8 | 0 | 10 |
| | 2 | 0 | 10 |
| 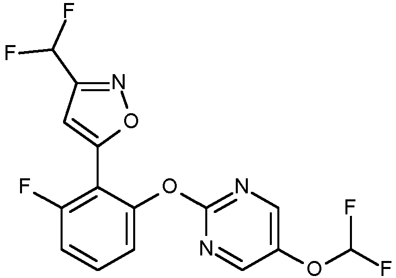 Compound 1.001 of the present invention | 500 | 40 | 100 |
| | 250 | 20 | 90 |
| | 125 | 10 | 80 |
| | 60 | 20 | 70 |
| | 8 | 0 | 20 |
| | 2 | NT | 30 |

**Table B3c**

| **Compound** | **Rate (g/ha)** | **ZEAMX** | **IPOHE** |
|---|---|---|---|
| 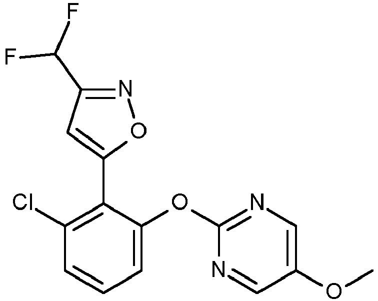 Comparator compound of the type disclosed in WO2015/108779 | 500 | NT | NT |
| | 250 | 30 | 60 |
| | 125 | 20 | 50 |
| | 60 | 10 | 20 |
| | 8 | 0 | 30 |
| | 2 | 0 | 20 |
| 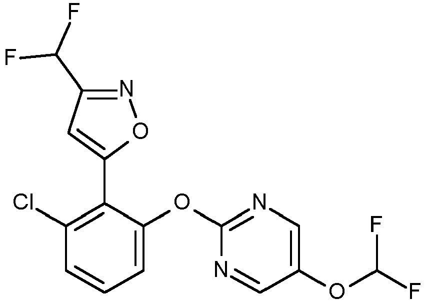 Compound 1.004 of the present invention | 500 | 40 | 90 |
| | 250 | 20 | 90 |
| | 125 | 10 | 70 |
| | 60 | 0 | 70 |
| | 8 | 0 | 50 |
| | 2 | 0 | 20 |

**Table B3d**

| **Compound** | **Rate (g/ha)** | **ZEAMX** | **IPOHE** |
|---|---|---|---|
| 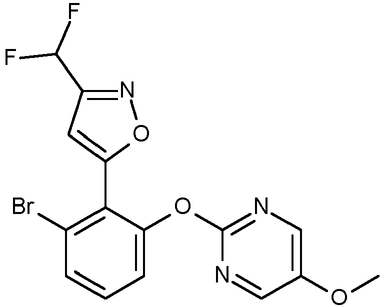 Comparator compound of the type disclosed in WO2015/108779 | 500 | NT | NT |
| | 250 | 30 | 60 |
| | 125 | 10 | 50 |
| | 60 | 0 | 50 |
| | 8 | 0 | 30 |
| | 2 | 0 | 10 |
| 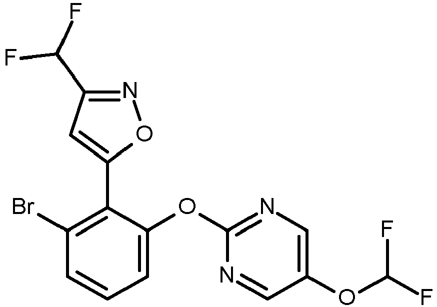 Compound 1.005 of the present invention | 500 | 30 | 80 |
| | 250 | 30 | 90 |
| | 125 | 20 | 70 |
| | 60 | 10 | 40 |
| | 8 | NT | 20 |
| | 2 | NT | 30 |

**Table B3e**

| **Compound** | **Rate (g/ha)** | **ZEAMX** | **IPOHE** |
|---|---|---|---|
| 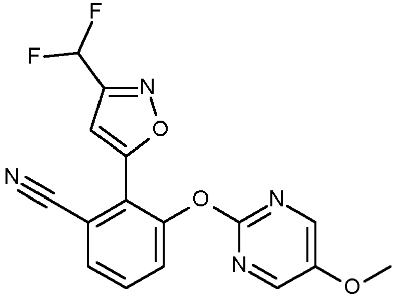 Comparator compound of the type disclosed in WO2015/108779 | 500 | 40 | 60 |
| | 250 | 10 | 50 |
| | 125 | 10 | 50 |
| | 60 | 0 | 40 |
| | 8 | 0 | 30 |
| | 2 | 0 | 10 |
| 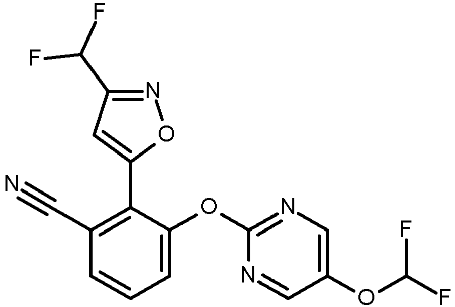 Compound 1.006 of the present invention | 500 | 20 | 80 |
| | 250 | 10 | 80 |
| | 125 | 10 | 70 |
| | 60 | 0 | 70 |
| | 8 | 0 | 60 |
| | 2 | 0 | 30 |

| | | | |
|---|---|---|---|
| NT = Not tested | | | |

The results from these comparative tests show that the compounds of the present invention provide surprisingly improved selective weed control. Thus, damage to a typical crop species (demonstrated using corn, ZEAMX) exhibited by the compounds of the present invention is similar or often reduced at a given application rate, while the control of weed species (demonstrated using *Ipomoea hederacea,* IPOHE) is unexpectedly improved.

## Claims

1. A compound of Formula (I): 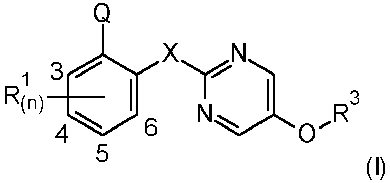 wherein
Q is selected from the group consisting of C₁-C₆haloalkyl, C₁-C₆haloalkyl-C(O)-, C₁-C₆haloalkoxy-, C₁-C₆haloalkoxyC(O)- and a 5-membered aromatic heterocyclic ring which is optionally substituted by 1 or 2 R² substituents independently selected from the group consisting of C₁-C₄alkyl, C₂-C₄alkenyl, C₂-C₄alkynyl, cyclopropyl, C₁-C₄haloalkyl, C₁-C₂alkoxy-, C₁-C₂haloalkoxy-, halogen, -C(O)C₁-C₄alkyl, NO₂, -CH₂CN, -CN and -S(O)ₚC₁-C₄alkyl;
each R¹ is independently selected from the group consisting of halogen, -CN, nitro, C₁-C₄alkyl, C₂-C₄alkenyl, C₂-C₄alkynyl, C₁-C₄haloalkyl, C₁-C₄alkoxy-, C₁-C₄haloalkoxy- and -S(O)ₚC₁-C₄alkyl;
R³ is C₁-C₂ haloalkyl;
X = O or S(O)ₚ;
n = 0, 1 or 2; and
p = 0, 1 or 2.

2. A compound according to claim 1, wherein Q is C₁-C₆fluoroalkyl.

3. A compound according to claim 1, wherein Q is C₁-C₆fluoroalkyl-C(O)-.

4. A compound of Formula (I) according to claim 1, wherein Q is a 5-membered aromatic heterocyclic ring selected from the group consisting of: 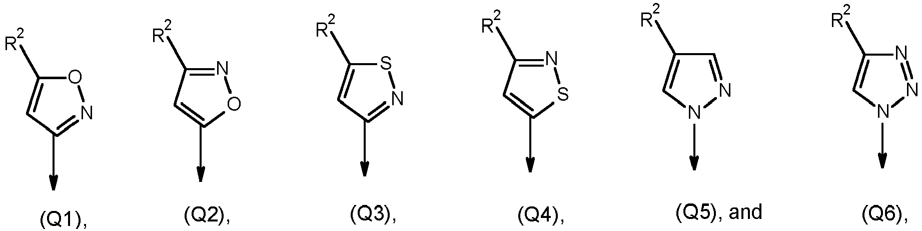 wherein R² is selected from the group consisting of hydrogen, C₁-C₄alkyl, C₂-C₄alkenyl, C₂-C₄alkynyl, cyclopropyl, C₁-C₂haloalkyl, C₁-C₂alkoxy-, C₁-C₂haloalkoxy-, halogen, -C(O)C₁-C₄alkyl, NO₂, -CH₂CN, -CN and -S(O)ₚC₁-C₄alkyl.

5. A compound of Formula (I) according to claim 4, wherein Q is selected from the group consisting of Q1, Q2 and Q5.

6. A compound of Formula (I) according to claim 4, wherein Q is Q2.

7. A compound according to any one of the previous claims, wherein n is 1 and R¹ is selected from the group consisting of fluoro, chloro, bromo and cyano.

8. A compound according to claim 7, wherein R¹ is selected from the group consisting of 3-fluoro, 3-chloro, 3-bromo and 3-cyano.

9. A compound according to any one of the previous claims, wherein R³ is CF₃ or CHF₂.

10. A herbicidal composition comprising a compound according to any one of the previous claims and an agriculturally acceptable formulation adjuvant.

11. A herbicidal composition according to claim 10, further comprising at least one additional pesticide.

12. A herbicidal composition according to claim 11, wherein the additional pesticide is a herbicide or herbicide safener.

13. A method of controlling weeds at a locus comprising application to the locus of a weed controlling amount of a composition according to any one of claims 10 to 12.

14. Use of a compound of Formula (I) as defined in claim 1 as a herbicide.

## Patentansprüche

1. Verbindung der Formel (I): 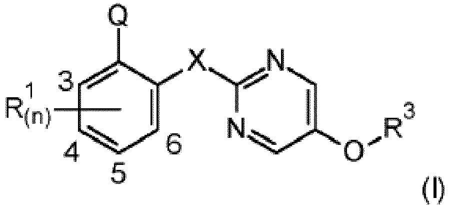 wobei
Q aus der Gruppe bestehend aus C₁-C₆-Halogenalkyl, C₁-C₆-Halogenalkyl-C(O)-, C₁-C₆-Halogenalkoxy-, C₁-C₆-Halogenalkoxy-C(O)- und einem 5-gliedrigen aromatischen heterocyclischen Ring, der gegebenenfalls durch 1 oder 2 R²-Substituenten, die unabhängig aus der Gruppe bestehend aus C₁-C₄-Alkyl, C₂-C₄-Alkenyl, C₂-C₄-Alkinyl, Cyclopropyl, C₁-C₄-Halogenalkyl, C₁-C₂-Alkoxy-, C₁-C₂-Halogenalkoxy-, Halogen, -C(O)-C₁-C₄-Alkyl, NO₂, -CH₂CN, -CN und -S(O)ₚ-C₁-C₄-Alkyl ausgewählt sind, substituiert ist, ausgewählt ist;
R¹ unabhängig aus der Gruppe bestehend aus Halogen, -CN, Nitro, C₁-C₄-Alkyl, C₂-C₄-Alkenyl, C₂-C₄-Alkinyl, C₁-C₄-Halogenalkyl, C₁-C₄-Alkoxy-, C₁-C₄-Halogenalkoxy- und - S(O)ₚC₁-C₄-Alkyl ausgewählt ist;
R³ für C₁-C₂-Halogenalkyl steht;
X = O oder S(O)ₚ;
n = 0, 1 oder 2; und
p = 0, 1 oder 2.

2. Verbindung nach Anspruch 1, wobei Q für C₁-C₆-Fluoralkyl steht.

3. Verbindung nach Anspruch 1, wobei Q für C₁-C₆-Fluoralkyl-C(O)- steht.

4. Verbindung der Formel (I) nach Anspruch 1, wobei Q für einen 5-gliedrigen aromatischen heterocyclischen 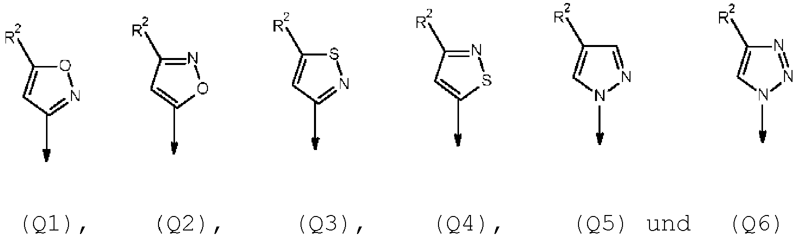 Ring steht, der aus der Gruppe bestehend aus:
ausgewählt ist, wobei R² aus der Gruppe bestehend aus Wasserstoff, C₁-C₄-Alkyl, C₂-C₄-Alkenyl, C₂-C₄-Alkinyl, Cyclopropyl, C₁-C₂-Halogenalkyl, C₁-C₂-Alkoxy-, C₁-C₂₋Halogenalkoxy-, Halogen, -C(O)-C₁-C₄-Alkyl, NO₂, -CH₂CN, -CN und -S(O)ₚ-C₁-C₄-Alkyl ausgewählt ist.

5. Verbindung der Formel (I) nach Anspruch 4, wobei Q aus der Gruppe bestehend aus Q1, Q2 und Q5 ausgewählt ist.

6. Verbindung der Formel (I) nach Anspruch 4, wobei Q für Q2 steht.

7. Verbindung nach einem der vorhergehenden Ansprüche, wobei n für 1 steht und R¹ aus der Gruppe bestehend aus Fluor, Chlor, Brom und Cyano ausgewählt ist.

8. Verbindung nach Anspruch 7, wobei R¹ aus der Gruppe bestehend aus 3-Fluor, 3-Chlor, 3-Brom und 3-Cyano ausgewählt ist.

9. Verbindung nach einem der vorhergehenden Ansprüche, wobei R³ für CF₃ oder CHF₂ steht.

10. Herbizide Zusammensetzung, umfassend eine Verbindung nach einem der vorhergehenden Ansprüche und ein landwirtschaftlich unbedenkliches Formulierungshilfsmittel.

11. Herbizide Zusammensetzung nach Anspruch 10, ferner umfassend mindestens ein zusätzliches Pestizid.

12. Herbizide Zusammensetzung nach Anspruch 11, wobei es sich bei dem zusätzlichen Pestizid um ein Herbizid oder einen Herbizid-Safener handelt.

13. Verfahren zur Bekämpfung von Unkräutern an einem Standort, bei dem man eine unkrautbekämpfende Menge einer Zusammensetzung nach einem der Ansprüche 10 bis 12 auf den Standort ausbringt.

14. Verwendung einer Verbindung der Formel (I) gemäß Anspruch 1 als Herbizid.

## Revendications

1. Composé de Formule (I) : 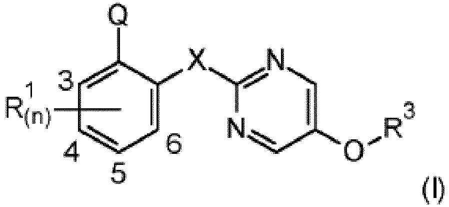
Q étant choisi dans le groupe constitué par C₁-C₆halogénoalkyle, C₁-C₆halogénoalkyl-C(O)-, C₁-C₆halogénoalcoxy-, C₁-C₆halogénoalcoxyC(O)- et un cycle hétérocyclique aromatique à 5 chaînons qui est éventuellement substitué par 1 ou 2 R² substituants indépendamment choisis dans le groupe constitué par C₁-C₄alkyle, C₂-C₄alcényle, C₂-C₄alcynyle, cyclopropyle, C₁-C₄halogénoalkyle, C₁-C₂alcoxy-, C₁-C₂halogénoalcoxy-, halogène, -C(O)C₁-C₄alkyle, NO₂, -CH₂CN, -CN et -S(O)ₚC₁-C₄alkyle ;
chaque R¹ étant indépendamment choisi dans le groupe constitué par halogène, -CN, nitro, C₁-C₄alkyle, C₂-C₄alcényle, C₂-C₄alcynyle, C₁-C₄halogénoalkyle, C₁-C₄alcoxy-, C₁-C₄halogénoalcoxy- et -S(O)ₚC₁-C₄alkyle ;
R³ étant C₁-C₂halogénoalkyle ;
X = O ou S(O)ₚ ;
n = 0, 1 ou 2 ; et
p = 0, 1 ou 2.

2. Composé selon la revendication 1, Q étant C₁-C₆fluoroalkyle.

3. Composé selon la revendication 1, Q étant C₁-C₆fluoroalkyl-C(O)-.

4. Composé de formule (I) selon la revendication 1, Q étant un cycle hétérocyclique aromatique à 5 chaînons choisi dans le groupe constitué par: 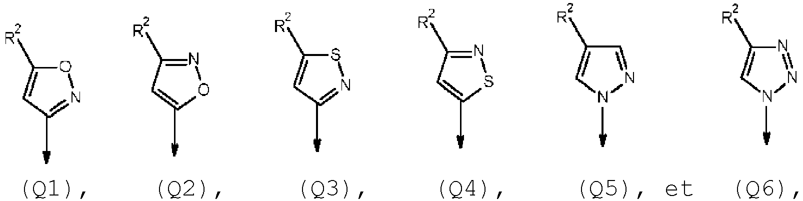 R² étant choisi dans le groupe constitué par hydrogène, C₁-C₄alkyle, C₂-C₄alcényle, C₂-C₄alcynyle, cyclopropyle, C₁-C₂halogénoalkyle, C₁-C₂alcoxy-, C₁-C₂halogénoalcoxy-, halogène, -C(O)C₁-C₄alkyle, NO₂, -CH₂CN, -CN et -S(O)pC₁-C₄alkyle.

5. Composé de formule (I) selon la revendication 4, Q étant choisi dans le groupe constitué par Q1, Q2 et Q5.

6. Composé de formule (I) selon la revendication 4, Q étant Q2.

7. Composé selon l'une quelconque des revendications précédentes, n étant 1 et R¹ étant choisi dans le groupe constitué par fluoro, chloro, bromo et cyano.

8. Composé selon la revendication 7, R¹ étant choisi dans le groupe constitué par 3-fluoro, 3-chloro, 3-bromo et 3-cyano.

9. Composé selon l'une quelconque des revendications précédentes, R³ étant CF₃ ou CHF₂.

10. Composition herbicide comprenant un composé selon l'une quelconque des revendications précédentes et un adjuvant de formulation acceptable sur le plan agricole.

11. Composition herbicide selon la revendication 10, comprenant en outre au moins un pesticide supplémentaire.

12. Composition herbicide selon la revendication 11, le pesticide supplémentaire étant un herbicide ou un phytoprotecteur herbicide.

13. Procédé de lutte contre des adventices au niveau d'un lieu comprenant une application sur le lieu d'une quantité de lutte contre des adventices d'une composition selon l'une quelconque des revendications 10 à 12.

14. Utilisation d'un composé de Formule (I) tel que défini selon la revendication 1, comme herbicide.
